# FASCICULE DE BREVET EUROPEEN

(11) **EP 0 646 368 B1**
(45) Date de publication et mention de la délivrance du brevet: **24.07.1996**
(21) Numéro de dépôt: 94402157.5
(22) Date de dépôt: 28.09.1994
(51) Int. Cl.: A61K 7/00, A61K 7/48, A61K 7/06

(54) **Composition d'organopolysiloxane d'aspect gélifié, sans gélifiant, utilisable en cosmétique et dermatologie**
Gel-ähnliche Zusammensetzung enthaltend Organopolysiloxane, ohne Geliermittel zur Kosmetische und Dermatologische Anwendung
A gel-resembling composition containing organopolysiloxane, without a gelling agent used in cosmetic and dermatology

(30) Priorité: 04.10.1993 FR 9311802
(43) Date de publication de la demande: 05.04.1995
(73) Titulaire: L'OREAL, F-75008 Paris (FR)
(72) Inventeur: Candau, Didier, F-91570 Bievres (FR); Simon, Pascal, F-94400 Vitry sur Seine (FR)
(74) Mandataire: Lhoste, Catherine

(56) Documents cités:
- EP-A- 0 458 600
- EP-A- 0 461 593
- WO-A-92/01508
- DE-A- 3 837 473
- DATABASE WPI Week 9343, Derwent Publications Ltd., London, GB; AN 93-339610 & JP-A-5 246 821 (SHISEIDO CO LTD) 24 Septembre 1993

## Description

La présente invention se rapporte à une composition d'organopolysiloxane d'aspect gélifié et brillant, sans gélifiant.

Cette composition constitue un excipient de choix pour l'incorporation de substances incompatibles avec les gélifiants classiquement utilisés dans les préparations d'aspect gélifié.

Cette composition peut servir de base pour la fabrication de crèmes trouvant application dans de nombreux domaines notamment cosmétiques, dermatologiques, pharmaceutiques, etc.

L'invention vise tout spécialement des crèmes de soin ou de maquillage de la peau, du visage, du corps en général y compris les ongles, le cuir chevelu et les cils.

Il existe déjà de nombreux produits tant cosmétiques que dermatologiques ou pharmaceutiques qui sont des émulsions huile dans eau (H/E). Par le choix des matières premières (émulsionnants, corps gras, polymères polyols et autres matières premières hydrophiles, etc.), ces dispersions peuvent se présenter sous la forme de crèmes de textures variées : plus ou moins épaisses, plus ou moins brillantes, plus ou moins gélifiées.

Pour obtenir une crème agréable d'un point de vue sensoriel (son "toucher") et visuel (son aspect), on recherche davantage des aspects gélifiés et brillants plutôt que inconsistants et mâts. Pour ce faire, on utilise habituellement un gélifiant (appelé aussi épaississant) que l'on incorpore dans la phase aqueuse continue de l'émulsion et qui donne sa consistance à la crème. La majorité des gélifiants utilisés classiquement sont des polymères carboxyvinyliques du type Carbomers (CTFA) que l'on neutralise par une base (soude, tri-éthanolamine).

Il arrive cependant que certaines matières premières, indispensables par ailleurs dans la composition de ces crèmes ne permettent pas l'utilisation des gélifiants cités précédemment par suite d'incompatibilité.

On sait par exemple que les électrolytes (sels inorganiques et organiques) sont incompatibles avec les polymères carboxyvinyliques neutralisés par le fait qu'ils "cassent" I'émulsion, voire la liquéfient.

Or un grand nombre de matières premières utilisées dans les émulsions H/E d'aspect gélifié sont soit des électrolytes à part entière, soit des mélanges de différents composés pouvant renfermer des quantités non négligeables d'électrolytes.

Ainsi des formulations contenant des polymères carboxyvinyliques et des électrolytes sont dépourvues de consistance et leur aspect les rend impropres à l'utilisation dans les domaines mentionnés ci-dessus.

Ce même problème se rencontre d'ailleurs avec tout composé, autre qu'un électrolyte, présentant une incompatibilité avec ces gélifiants carboxyvinyliques.

Une solution consiste à utiliser, à la place des gélifiants polymères carboxyvinyliques, des gélifants de type polysaccharidiques tels que gomme naturelle de cellulose, guar ou xanthane.

Mais pour obtenir une consistance équivalente à celle procurée par les gélifiants de type polymères carboxyvinyliques, on doit incorporer des quantités importantes de gélifiant pour accroitre la viscosité de l'émulsion, ce qui est gênant dans les applications mentionnées ci-dessus, car alors la crème d'aspect gélifié devient très collante lors de son application sur la peau, les cheveux, etc.

Il est par ailleurs connu selon le document US-A-5013715 de réaliser une base anhydre d'aspect pâteux et mat pour la préparation de crèmes cosmétiques. Cette base est constituée d'une huile organopolysiloxane fluide du type méthyl- phénylpolysiloxane utilisée seule ou en mélange avec un polydiméthylsiloxane (PDMS) cyclique et/ou un PDMS linéaire et d'ester de saccharose. Cet ester a un caractère lipophile marqué car son indice d'hydroxyle est inférieur à 200, pour être compatible avec l'organopolysiloxane fluide.

L'ajout d'eau dans cette pâte anhydre conduit à l'obtention d'une composition difficilement applicable sur la peau.

On sait également selon le document EP-A-158108 qu'il est possible de réaliser des émulsions H/E en ajoutant de l'eau à des compositions anhydres à visée détergente comprenant une phase huileuse, un agent émulsionnant détergent et un ester de saccharose.

Ces émulsions sont des crèmes laiteuses et peu visqueuses, difficiles à appliquer sur la peau et les cheveux.

En outre, il est connu du document EP-458600 des émulsions contenant des esters de saccharose, permettant l'obtention de compositions occlusives qui limitent la perte en eau de la peau. Ces émulsions contiennent, par ailleurs, un émulsionnant et, quand il s'agit d'émulsions huile-dans-eau, un polymère carboxyvinylique comme gélifiant.

Il subsiste donc le besoin de compositions gélifiées ne présentant pas les inconvénients rencontrés avec les préparations connues notamment d'inconsistance, d'instabilité, d'aspect mat, de sensation désagréable au toucher, d'effet collant, d'inaptitude à l'étalement et d'incompatibilité avec certains composés.

La demanderesse a maintenant trouvé une composition d'aspect gélifié, sans gélifiant, permettant de remédier à ces inconvénients.

Cette composition comprend une phase huileuse, formée d'au moins 75 % d'organopolysiloxane et dispersée dans une phase aqueuse ayant au moins 25 % d'eau à l'aide d'un émulsionnant formé uniquement d'au moins un ester de saccharose et d'acide gras.

Dans la présente invention, on entend par composition "d'aspect gélifié" tout type de produit crêmeux d'une certaine consistance, ne s'écoulant pas sous son propre poids et ayant une viscosité supérieure ou égale à 1 Pa.s.

L'intérêt de cette composition huile dans eau est de permettre l'introduction d'un ou plusieurs additifs hydrophiles dans la phase aqueuse ayant des propriétés actives d'un point de vue cosmétique et/ou dermatologique et/ou pharmaceutique, sans modifier sa texture gélifiée.

Ces actifs hydrophiles sont principalement des électrolytes. On entend par "électrolyte" un sel organique ou inorganique d'acide organique ou minéral et d'une base organique ou minérale.

Aussi, la composition de l'invention comprend avantageusement, en poids :
- de 5 % à 50 % de phase huileuse
- de 1 % à 20 % d'ester de saccharose
- de 30 % à 94 % de phase aqueuse
- de 0 % à 5 % d'additif.

De préférence encore, cette composition comprend, en poids :
- de 15 % à 35 % de phase huileuse
- de 3 % à 8 % d'ester de saccharose
- de 54 % à 81 % de phase aqueuse
- de 0 % à 5 % d'additif.

Selon l'invention, l'électrolyte ou le mélange d'électrolytes est utilisé à raison de 0,01 % à 1 % en poids par rapport au poids total de la composition.

De façon surprenante, l'aspect gélifié de la composition de l'invention n'est pas perturbé par l'introduction d'électrolyte ou de toute matière incompatible avec les gélifiants classiques du type carboxyvinyliques comme les sels d'ammonium et d'imidazolinium, certains conservateurs tels que le di-iséthionate du di-(amidino-4-phénoxy)-1,6 hexane.

Les actifs hydrophiles tels que les électrolytes sont choisis dans le groupe comprenant des agents hydratants, filtrants anti UV-A, anti UV-B, anti UV-C, kératolfliques, antivieillissement, antirides, anti-oxydants, dépigmentants, liporégulateurs, anti-inflammatoires, rafraichissants, cicatrisants, antibactériens, antifongiques, antitranspirants, antipelliculaires, réducteurs pour permanentes, conditionneurs pour cheveux, nourrissants, filmogènes (restructurants, tenseurs, plastifiants).

De manière préférée, l'électrolyte est choisi parmi les sels de sodium comme le pyrrolidone carboxylate de sodium, jouant le rôle d'hydratant, le sel de sodium de l'acide 3,3'-(1,4-phénylènediméthylidyne)bis[7.7-diméthyl-2-oxo-bicyclo[2.2.1.]heptane-1-méthanesulfonique], servant de filtres solaires anti U.V.A., les sels d'imidazolinium ou de magnésium, les sels de l'acide salicylique et de ses dérivés tels que ceux cités dans le document EP-A-378936, servant d'agents antivieillissement et les sels d'alpha-hydroxyacides tels que ceux décrits dans le document US-A-4380549, ou dans le document WO 92/18116, servant pour le soin de la peau flétrie ou pour retarder son vieillissement, comme les sels d'ammonium.

Selon un aspect préféré de l'invention, l'organopolysiloxane est choisi dans le groupe comprenant les cyclodiméthylsiloxanes tels que, par exemple, le cyclopentadiméthylsiloxane, les polydiméthylsiloxanes de viscosité différente tels que, par exemple, les DC fluid 200 cSt à 12 500 cSt (2.10⁻²m²/s à 1,2 m²/s) vendus par DOW CORNING ou l'Abil 10 vendu par GOLDSCHMIDT, les polyalcoxysiloxanes tels que, par exemple, le polystéaroxydiméthylsiloxane Abil Wax 2434 vendu par GOLDSCHMIDT, les polyalkylsiloxanes tels que, par exemple, le polycétylméthylsiloxane Abil Wax 9801 vendu par GOLDSCHMIDT, les polyhydroxysiloxanes tels que, par exemple, le Q2-1403 Fluid de DOW CORNING, les polyphénylsiloxanes tels que, par exemple, le polyphénylméthylsiloxane Silbione 70641 V200 vendu par RHÔNE-POULENC ou le polyphényltriméthylsiloxane Abil AV 20 de GOLDSCHMIDT.

De façon générale, le groupement alkyle ou alcoxy des organopolysiloxanes a de 12 à 22 atomes de carbone.

Le reste de la phase huileuse comporte tout type d'huile minérale, organique, végétale ou synthétique. Les huiles utilisées sont celles connues de l'homme du métier telles que le beurre de karité (voir le document US-A-4661343), l'huile d'amande, d'abricot, le perhydrosqualène de synthèse, les esters d'acides gras et d'alcools en C1 à C30 tels que le palmitate d'éthyl-2 hexyle, le myristate d'isopropyle.

Selon un autre aspect préféré de l'invention, l'ester de saccharose est choisi dans le groupe comprenant les esters d'acide gras en C7 à C22 linéaires ou ramifiés, saturés ou insaturés et de saccharose et miaux en C12 à C22, lesdits acides gras étant par exemple fixés sur le motif saccharose en position C2 et/ou C3 et/ou C5 et/ou C6 du résidu fructose et/ou en position C2 et/ou C3 et/ou C4 et/ou C6 du résidu glucose.

De préférence ces esters seront choisis parmi les mono-, di-, tri- et tétraesters et les polyesters comprenant des monoesters à raison de 30 % à 95 % en poids par rapport au poids total d'ester.

De façon avantageuse, l'ester de saccharose a un indice d'hydroxyle lui confèrant un caractère hydrophile marqué.

Les esters de saccharose conformes à l'invention sont par exemple ceux vendus par la société CRODESTA sous les dénominations F160, F140, F110, F90, F70, SL40, désignant respectivement les palmitostéarates de saccharose formés de 73 % monoester et 27 % di-,triester, de 61 % monoester et 39 % di-tri-tétraester, de 52 % monoester et 48 % di-tri-tétraester, de 45 % monoester et de 55 % di-tri-tétraester, de 39 % monoester et de 61 % de di-, tritétraester, le monolaurate de saccharose. On peut aussi mélanger ces différents produits à différents pourcentages pour obtenir la consistance souhaitée.

On peut aussi utiliser ceux vendus par la société MITSUBISHI sous la dénomination Ryoto Sugar esters par exemple sous la référence B370 et correspondant au béhénate de saccharose formé de 20 % monoester et 80 % di-triester-polyester. Ce composé est préférentiellement combiné aux esters de saccharose décrits précédemment.

La composition selon l'invention peut également comprendre un ou plusieurs additifs autres qu'un électrolyte.

Cet additif peut être choisi dans le groupe comprenant des agents bien connus de l'homme du métier que sont par exemple les coémulsionnants tels que les alcools gras en C₁₂ à C₂₂ ou des mono-, di-glycérides, des stabilisants d'émulsion tels que les polymères polysaccharidiques à faible dose (0,1 % à 0,3 %), des antioxydants (les vitamines ou leurs esters), des conservateurs, des colorants, des pigments, des parfums, des hydratants tels que les polyols (glycérine, sorbitol) et les glycols (butylène ou hexylène glycol), des acides aminés et leurs dérivés, etc.

On utilise ces additifs aux concentrations communément admises pour chacun d'entre eux notamment selon son degré de solubilité. De manière générale, les additifs cités peuvent être utilisés à raison de 0,05 % à 5 %.

Selon un aspect particulièrement préféré de l'invention, la composition d'aspect gélifié, sans gélifiant consiste, en poids :
- de 5 % à 50 % d'une phase huileuse, constituée d'au moins 75 % d'organopolysiloxane, dispersée dans une phase aqueuse
- de 1 % à 20 % d'ester de saccharose servant d'émulsionnant
- de 30 % à 93,9 % de phase aqueuse
- de 0,1 % à 1 % d'un électrolyte formé du sel de sodium de l'acide 3,3'-(1,4-phénylènediméthylidyne)bis[7.7-diméthyl-2-oxobicyclo[2.2.1.]heptane-1-méthanesulfonique].

L'invention se rapporte aussi à une utilisation de la composition décrite précédemment pour le traitement cosmétique de la peau, des ongles, du cuir chevelu et des cils.

L'invention a encore pour objet l'utilisation de cette composition pour l'obtention d'une crème destinée au traitement dermatologique de la peau et du cuir chevelu.

L'invention va être maintenant plus complètement décrite dans ses objets et ses caractéristiques à l'aide des exemples et contre exemples qui vont suivre, donnés à titre non limitatif.

Dans les exemples on met en oeuvre le mode opératoire suivant : on incorpore tout d'abord l'ester de saccharose dans de l'eau contenant éventuellement des additifs hydrophiles (par exemple des électrolytes), puis on chauffe entre 60°C et 70°C sous agitation moyenne pendant 15 min. à 45 min. Quand l'ester est bien dissout on réalise l'émulsion par introduction dans la phase aqueuse de la phase huileuse préalablement chauffée à 60°C, contenant éventuellement des additifs lipophiles, puis on agite violemment par cisaillement à l'aide d'un mélangeur de type "Moritz" pendant 5 min à 15 min, en maintenant le chauffage. Enfin, on laisse revenir à température ambiante sous agitation modérée (à l'aide d'un mélangeur à palettes) pour dégazer l'émulsion.

Le résultat est une crème d'aspect gélifié et brillant et de viscosité supérieure à 1 Pa.s.

Les compositions ci-après sont données en pourcentage pondéral.

### EXEMPLE 1

| | |
|---|---|
| Palmito-stéarate de saccharose - CRODESTA F70 (45 % mono-ester, 55 % di-tri-ester) | 6 |
| Alcool béhénique (co-émulsionnant) | 2,5 |
| Polydiméthylsiloxane (DOW CORNING Fluid 200 - 10 cSt) | 25 |
| BHA, BHT (anti-oxydants) | 0,8 |
| Parahydroxybenzoate de méthyle (conservateur) | 0,4 |
| Eau | qsp 100 |

La crème obtenue dépourvue de gélifiant est non collante, agréable, et destinée aux peaux à tendance grasse.

### EXEMPLE 2

| | |
|---|---|
| Palmito-stéarate de saccharose CRODESTA F70 (45 % mono-ester, 55 % di-tri-ester) | 8 |
| Polydiméthylsiloxane (DOW CORNING Fluid 200 - 200 cSt) | 7 |
| Cyclopentadiméthylsiloxane (DOW CORNING Fluid 245) | 5 |
| Polystéaroxydiméthylsiloxane (Abil Wax 2434- GOLDSCHMIDT) | 3 |
| Beurre de karité liquide (huile) | 3 |
| BHA, BHT | 0,4 |
| Imidazolidinylurée (conservateur) | 0,05 |
| Eau | qsp 100 |

La crème obtenue est brillante et gélifiée, facilement applicable sur la peau et sert au soin des peaux sèches.

### EXEMPLE 3

| | |
|---|---|
| Palmito-stéarate de saccharose CRODESTA F50 (20 % mono-ester, 80 % di-tri-polyester) | 4 |
| Laurate de saccharose SL40 CRODESTA | 4 |
| Alcool cétylique (co-émulsionnant) | 0,5 |
| 13 % polydiméthylsiloxanol - 87 % polydiméthylsiloxane 5 cSt (DOW CORNING Q2 - 1403 Fluid) | 4 |
| Polyphénylméthylsiloxane (Silbione 70641 V200 - RHONE POULENC) | 15 |
| Cyclopentadiméthylsiloxane | 20 |
| BHA, BHT | 0,2 |
| Parahydroxybenzoate de méthyle | 0,1 |
| Eau | qsp 100 |

Cette crème d'aspect gélifié et brillant sert au soin des peaux sèches.

### EXEMPLE 4

| | |
|---|---|
| Béhénate de saccharose (MITSUBISHI Ryoto sugar esters B370) (20 % mono-ester, 80 % di-tri-polyester) | 2 |
| Palmito-stéarate de saccharose CRODESTA F160 (73 % mono-ester, 27 % di-tri-ester) | 2,5 |
| 67 % polydiméthylsiloxane - 33 % triméthylsiloxisilicate (DOW CORNING Fluid 200 - 100 cSt) | 3 |
| Polydiméthylsiloxane (DOW CORNING Fluid 200 - 100 cSt) | 10 |
| Palmitate d'éthyl - 2 hexyle (huile) | 5 |
| Chlorure d'hydroxypropylammonium guar (stabilisant) | 0,1 |
| BHA, BHT | 0,4 |
| Imidazolidinylurée | 0,25 |
| Eau | qsp 100 |

### EXEMPLE 5

Dans la composition selon l'exemple 1 on ajoute un électrolyte formé du pyrrolidone carboxylate de sodium à une teneur de 1 % en poids par rapport à la composition totale. On obtient une crême d'aspect gélifié, destinée à l'hydratation de la peau.

### CONTRE-EXEMPLE

Dans un gel aqueux contenant 0,5 % du polymère carboxyvinylique dénommé "Carbopol" vendu par la société GOODRICH, neutralisé à la soude, on ajoute du NaCl ou l'électrolyte de l'exemple 5 dans les mêmes proportions. On constate que la composition est entièrement fluidifée et a une viscosité proche de celle de l'eau. De ce fait elle ne correspond plus à ce qu'on recherche.

### EXEMPLE 6

Dans la composition de l'exemple 1 on ajoute 0,7 % d'un filtre anti-UVA qui est le sel de sodium de l'acide 3,3'-(1,4-phénylènediméthylidyne)bis[7.7-diméthyl-2-oxobicyclo[2.2.1.]heptane-1-méthanesulfonique].

On réalise ainsi une crème de protection solaire d'aspect gélifié et brillant.

### EXEMPLE 7

Dans la composition selon l'exemple 2, on remplace le conservateur utilisé par le di-isethionate du di(amidino-4-phénoxy)-1,6-hexane. L'aspect de la crème reste inchangé.

## Revendications

1. Composition d'aspect gélifié, sans gélifiant, comprenant une phase huileuse formée d'au moins 75 % d'organopolysiloxane, dispersée dans une phase aqueuse ayant au moins 25 % d"eau à l'aide d'un émulsionnant formé uniquement d'au moins un ester de saccharose et d'acide gras.

2. Composition selon la revendication 1, caractérisée en ce que l'ester de saccharose est choisi dans le groupe comprenant les esters d'acide gras en C7 à C22 linéaires ou ramifiés, saturés ou insaturés et de saccharose.

3. Composition selon la revendication 1 ou 2, caractérisée en ce que l'ester de saccharose est choisi dans le groupe comprenant les esters d'acide gras en C12 à C22 linéaires ou ramifiés, saturés ou insaturés et de saccharose.

4. Composition selon l'une quelconque des revendications précédentes, caractérisée en ce que l'ester est choisi parmi les esters mono-, di-, tri-, et tétra-esters et les polyesters comprenant les monoesters à raison de 30% à 95% en poids par rapport au poids total d'ester.

5. Composition selon l'une quelconque des revendications précédentes, caractérisée en ce qu'elle contient un électrolyte.

6. Composition selon l'une quelconque des revendications précédentes, caractérisée en ce qu'elle contient un électrolyte choisi parmi :
- le pyrrolidone carboxylate de sodium
- le sel de sodium de l'acide 3,3'-(1,4-phénylènediméthylidyne)bis [7.7-diméthyl-2-oxobicyclo[2.2.1]heptane-1-méthanesulfonique]
- les sels de l'acide salicylique et de ses dérivés
- les sels d'alphahydroxyacides.

7. Composition selon la revendication 5 ou 6, caractérisée en ce que l'électrolyte est utilisé à raison de 0,01 % à 1 % en poids.

8. Composition selon l'une quelconque des revendications précédentes, caractérisée en ce que l'organopolysiloxane est choisi dans le groupe comprenant les cyclodiméthylsiloxanes, polydiméthylsiloxanes, polyalcoxysiloxanes, polyalkylsiloxanes, polyhydroxysiloxanes, polyphénylsiloxanes.

9. Composition selon l'une quelconque des revendications précédentes, caractérisée en ce qu'elle contient au moins un additif autre qu'un électrolyte.

10. Composition selon la revendication 8, caractérisée en ce que l'additif autre qu'un électrolyte est choisi dans le groupe comprenant des agents coémulsionnants, stabilisants, antioxydants, conservateurs, colorants, pigments, parfumants.

11. Composition selon la revendication 9 ou 10, caractérisée en ce que l'additif comprend un polysaccharide d'origine naturelle à une concentration allant de 0,1 % à 0,3 % en poids par rapport au poids total de la composition.

12. Composition selon l'une quelconque des revendications précédentes, caractérisée en ce qu'elle comprend, en poids :
- de 5 % à 50 % de phase huileuse
- de 1 % à 20 % d'ester de saccharose
- de 30 % à 94 % de phase aqueuse
- de 0 % à 5 % d'additif.

13. Composition selon l'une quelconque des revendications précédentes, caractérisée en ce qu'elle comprend, en poids :
- de 15 % à 35 % de phase huileuse
- de 3 % à 8 % d'ester de saccharose
- de 54 % à 81 % de phase aqueuse
- de 0 % à 5 % d'additif.

14. Composition d'aspect gélifié, sans gélifiant, selon la revendication 1 comprenant en poids :
- de 5% à 50 % d'une phase huileuse, constituée d'au moins 75 % d'organopolysiloxane, dispersée dans une phase aqueuse
- de 1 % à 20 % d'ester de saccharose servant d'émulsionnant
- de 30 % à 93,9 % de phase aqueuse
- de 0,1 % à 1 % d'un electrolyte formé du sel de sodium de l'acide 3,3'-(1,4- phénylènediméthylidyne)bis[7.7-diméthyl-2-oxobicyclo[2.2.1.]heptane-1-méthanesulfonique].

15. Utilisation d'une composition selon l'une quelconque des revendications précédentes pour le traitement cosmétique de la peau, des ongles, du cuir chevelu et/ou des cils.

16. Utilisation d'une composition selon l'une quelconque des revendications 1 à 14 pour l'obtention d'une crème destinée au traitement dermatologique de la peau et du cuir chevelu.

## Claims

1. Composition of gel-like appearance, containing no gelling agent, comprising an oily phase formed of at least 75 % of organopolysiloxane, dispersed in an aqueous phase containing at least 25 % of water, using an emulsifying agent formed exclusively of at least one ester of sucrose and fatty acid.

2. Composition according to Claim 1, characterized in that the sucrose ester is chosen from the group comprising saturated or unsaturated, linear or branched C7 to C22 fatty acids esters of sucrose.

3. Composition according to Claim 1 or 2, characterized in that the sucrose ester is chosen from the group comprising saturated or unsaturated, linear or branched C12 to C22 fatty acids esters of sucrose.

4. Composition according to any one of the preceding claims, characterized in that the ester is chosen from the mono-, di-, tri- and tetraesters and the polyesters comprising the monoesters in an amount of 30 % to 95 % by weight relative to the total weight of ester.

5. Composition according to any one of the preceding claims, characterized in that it contains an electrolyte.

6. Composition according to any one of the preceding claims, characterized in that it contains an electrolyte chosen from:
- sodium pyrrolidonecarboxylate
- the sodium salt of 3,3'-(1,4-phenylenedimethylidyne)- bis [7,7-dimethyl-2-oxobicyclo[2.2.1]heptane-1-methanesulphonic] acid
- salicylic acid salts and derivatives thereof
- alpha-hydroxy acid salts.

7. Composition according to Claim 5 or 6, characterized in that the electrolyte is used in an amount of 0.01 % to 1 % by weight.

8. Composition according to any one of the preceding claims, characterized in that the organopolysiloxane is chosen from the group comprising cyclodimethylsiloxanes, polydimethylsiloxanes, polyalkoxysiloxanes, polyalkylsiloxanes, polyhydroxysiloxanes and polyphenylsiloxanes.

9. Composition according to any one of the preceding claims, characterized in that it contains at least one additive other than an electrolyte.

10. Composition according to Claim 8, characterized in that the additive other than an electrolyte is chosen from the group comprising co-emulsifying agents, stabilizing agents, antioxidants, preserving agents, dyes, pigments and fragrances.

11. Composition according to Claim 9 or 10, characterized in that the additive comprises a polysaccharide of natural origin in a concentration ranging from 0.1 % to 0.3 % by weight relative to the total weight of the composition.

12. Composition according to any one of the preceding claims, characterized in that it comprises, by weight:
- from 5 % to 50 % of oily phase
- from 1 % to 20 % of sucrose ester
- from 30 % to 94 % of agueous phase
- from 0 % to 5 % of additive.

13. Composition according to any one of the preceding claims, characterized in that it comprises, by weight:
- from 15 % to 35 % of oily phase
- from 3 % to 8 % of sucrose ester
- from 54 % to 81 % of aqueous phase
- from 0 % to 5 % of additive.

14. Composition of gel-like appearance, containing no gelling agent, according to Claim 1, comprising by weight:
- from 5 % to 50 % of an oily phase, consisting of at least 75 % of organopolysiloxane, dispersed in an aqueous phase
- from 1 % to 20 % of sucrose ester serving as an emulsifying agent
- from 30 % to 93.9 % of aqueous phase
- from 0.1 % to 1 % of an electrolyte formed of the sodium salt of 3,3'-(1,4-phenylenedimethylidyne)bis[7,7-dimethyl-2-oxobicyclo[2.2.1]heptane-1-methanesulphonic] acid.

15. Use of a composition according to any one of the preceding claims for the cosmetic treatment of the skin, the nails, the scalp and/or the eyelashes.

16. Use of a composition according to any one of Claims 1 to 14 in order to obtain a cream intended for the dermatological treatment of the skin and of the scalp.

## Patentansprüche

1. Gelartige Zusammensetzung ohne Gelbildner, die eine aus mindestens 75 % Polyorganosiloxan bestehende Ölphase enthält, die in einer wäßrigen Phase, die mindestens 25 % Wasser enthält, mit Hilfe eines Emulgators dispergiert ist, der lediglich aus mindestens einem Fettsäure-Saccharoseester besteht.

2. Zusammensetzung nach Anspruch 1, dadurch gekennzeichnet, daß der Saccharoseester unter Estern von geradkettigen oder verzweigten gesättigten oder ungesättigten C₇₋₂₂-Fettsäuren mit Saccharose ausgewählt ist.

3. Zusammensetzung nach Anspruch 1 oder 2, dadurch gekennzeichnet, daß der Saccharoseester unter Estern von geradkettigen oder verzweigten gesättigten oder ungesättigten C₁₂₋₂₂-Fettsäuren mit Saccharose ausgewählt ist.

4. Zusammensetzung nach einem der vorhergehenden Ansprüche, dadurch gekennzeichnet, daß der Ester unter Mono-, Di-, Tri- und Tetraestern sowie Polyestern ausgewählt ist, die 30 bis 95 Gew.-% Monoester, bezogen auf das Gesamtgewicht der Ester, enthalten.

5. Zusammensetzung nach einem der vorhergehenden Ansprüche, dadurch gekennzeichnet, daß sie einen Elektrolyten enthält.

6. Zusammensetzung nach einem der vorhergehenden Ansprüche, dadurch gekennzeichnet, daß sie einen Elektrolyten enthält, der ausgewählt ist unter:
- Natriumpyrrolidoncarboxylat,
- dem Natriumsalz von 3,3-(1,4-Phenylendimethylidin) bis(7,7-dimethyl-2-oxobicyclo[2.2.1]heptan-1-methansulfonsäure),
- Salzen von Salicylsäure und ihren Derivaten und
- Salzen von α-Hydroxysäuren.

7. Zusammensetzung nach Anspruch 5 oder 6, dadurch gekennzeichnet, daß der Elektrolyt in einem Mengenanteil von 0,01 bis 1 Gew.-% eingesetzt ist.

8. Zusammensetzung nach einem der vorhergehenden Ansprüche, dadurch gekennzeichnet, daß das Polyorganosiloxan unter Cyclodimethylsiloxanen, Polydimethylsiloxanen, Polyalkoxysiloxanen, Polyalkylsiloxanen, Polyhydroxysiloxanen und Polyphenylsiloxanen ausgewählt ist.

9. Zusammensetzung nach einem der vorhergehenden Ansprüche, dadurch gekennzeichnet, daß sie mindestens ein Additiv enthält, das kein Elektrolyt ist.

10. Zusammensetzung nach Anspruch 8, dadurch gekennzeichnet, daß das Additiv, das kein Elektrolyt ist, unter Coemulgatoren, Stabilisierungsmitteln, Antioxidantien, Konservierungsmitteln, Färbemitteln, Pigmenten und Parfumierungsmitteln ausgewählt ist.

11. Zusammensetzung nach Anspruch 9 oder 10, dadurch gekennzeichnet, daß das Additiv ein Polysaccharid natürlichen Ursprungs ist, das in einer Konzentration von 0,1 bis 0,3 Gew.-%, bezogen auf das Gesamtgewicht der Zusammensetzung, vorliegt.

12. Zusammensetzung nach einem der vorhergehenden Ansprüche, dadurch gekennzeichnet, daß sie folgende Bestandteile enthält, deren Mengenanteile gewichtsbezogen sind:
- 5 bis 50 % Ölphase,
- 1 bis 20 % Saccharoseester,
- 30 bis 94 % wäßrige Phase und
- 0 bis 5 % Additive.

13. Zusammensetzung nach einem der vorhergehenden Ansprüche, dadurch gekennzeichnet, daß sie folgende Bestandteile enthält, deren Mengenanteile gewichtsbezogen sind:
- 15 bis 35 % Ölphase,
- 3 bis 8 % Saccharoseester,
- 54 bis 81 % wäßrige Phase und
- 0 bis 5 % Additive.

14. Gelartige Zusammensetzung, die keinen Gelbildner enthält, nach Anspruch 1, die folgende Bestandteile enthält, deren Mengenanteile gewichtsbezogen sind:
- 5 bis 50 % einer Ölphase, die aus mindestens 75 % Polyorganosiloxan besteht und in einer wäßrigen Phase dispergiert ist,
- 1 bis 20 % Saccharoseester, der als Emulgator dient,
- 30 bis 93,9 % wäßrige Phase und
- 0,1 bis 1 % eines Elektrolyten aus dem Natriumsalz von 3,3'-(1,4-Phenylendimethylidin)-bis(7,7-dimethyl-2-oxobicyclo[2.2.1]heptan-1-methansulfon säure).

15. Verwendung einer Zusammensetzung nach einem der vorhergehenden Ansprüche zur kosmetischen Behandlung der Haut, der Nägel, der Kopfhaut und/oder der Wimpern.

16. Verwendung einer Zusammensetzung nach einem der Ansprüche 1 bis 14 zur Herstellung einer Creme, die zur dermatologischen Behandlung der Haut und der Kopfhaut bestimmt ist.
